(19) Europäisches Patentamt
European Patent Office
Office européen des brevets

(11) Publication number: **0 171 739**
**B1**

(12) **EUROPEAN PATENT SPECIFICATION**

(45) Date of publication of patent specification: **10.01.90**

(21) Application number: **85109896.2**

(22) Date of filing: **06.08.85**

(51) Int. Cl.[5]: **C 07 D 487/04,** A 61 K 31/505 // C07D333/38, C07D409/12, C07D413/12, C07D405/12, C07D401/12 ,(C07D487/04, 239:00, 235:00)

(54) **Imidazo[1,5-a]pyrimidine derivatives and process for their preparation.**

(30) Priority: **07.08.84 JP 165468/84**

(43) Date of publication of application:
**19.02.86 Bulletin 86/08**

(45) Publication of the grant of the patent:
**10.01.90 Bulletin 90/02**

(84) Designated Contracting States:
**AT BE CH DE FR GB IT LI NL SE**

(56) References cited:
**EP-A-0 134 928**
**DE-A-2 160 020**

(73) Proprietor: **KYORIN PHARMACEUTICAL CO., LTD.**
**No. 5, Kanda Surugadai 2-chome**
**Chiyoda-ku Tokyo (JP)**

(72) Inventor: **Irikura, Tsutomu**
**10-28, Ooizumigakuen-cho**
**7-chome Nerima-ku Tokyo (JP)**
Inventor: **Suzue, Seigo**
**No. 13-4, Aoba 4-chome**
**Kuki-shi Saitama-ken (JP)**
Inventor: **Murayama, Satoshi**
**No. 6095, Ooaza Tomonuma Nogi-cho**
**Shimotsuga-gun Tochigi-ken (JP)**
Inventor: **Kinoshita, Susumu**
**No. 7-6, Kawagashi Naka 2-chome**
**Okaya-shi Nagano-ken (JP)**

(74) Representative: **Patentanwälte TER MEER - MÜLLER - STEINMEISTER**
**Mauerkircherstrasse 45**
**D-8000 München 80 (DE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a new class of compound of imidazo[1,5-*a*]pyrimidine series. It relates also to the synthesis of such substance. It is concerned further with salts of these compounds such as hydrochloride, sulfate, acetate, tartarate and methanesulfonate.

The EP—A—0 134 928 relates to imidazo[1,5-*a*]pyrimidine derivatives having substituents representing a hydrogen atom, halogen atom or an alkyl group. Further on it is described that these compounds possess valuable pharmacological properties, i.e. antifungal effects.

Recently, fungal diseases are on the increase internationally because of frequent use of broad spectrum antibiotics, steroid hormones and immunosuppressive agent et cetera.

However, useful antifungal agents in therapy of fungal diseases are limited. At present, it might almost be said that the drug for fungal diseases is polyenmacrolide and imidazole derivatives. It has been expected to develop more useful antifungal agents for treatment of fungal disease. Therefore, we have studied earnestly to develop compounds having useful activity, especially great potent antifungal activity.

As a result of our study, we have found novel imidazo[1,5-a]pyrimidine derivatives having different structure and high potency against various organisms as compared with known antifungal agents.

The present invention relates to new imidazo[1,5-*a*]pyrimidine derivatives, namely:

2,4-dichloro-6-(2-thienyl)imidazo[1,5-a]pyrimidine,
2,4-dichloro-6-(5-phenyl-3-isoxazolyl)imidazo[1,5-a]pyrimidine,
2,4-dichloro-6-(2-furyl)imidazo[1,5-a]pyrimidine,
2,4-dichloro-6-(2-thienylmethyl)imidazo[1,5-a]pyrimidine,
2,4-dichloro-6-(3-thienyl)imidazo[1,5-a]pyrimidine,
2,4-dichloro-6-(5-bromo-2-thienyl)imidazo[1,5-a]pyrimidine,
2,4-dichloro-6-(5-chloro-2-thienyl)imidazo[1,5-a]pyrimidine,
2,4-dichloro-6-(5-methyl-2-thienyl)imidazo[1,5-a]pyrimidine,
2,4-dichloro-6-(3-pyridyl)imidazo[1,5-a]pyrimidine,
2,4-dichloro-6-(3-chloro-1-benzothiophen-2-yl)imidazo[1,5-a]pyrimidine,
2,4-dichloro-6-phenylthiomethylimidazo[1,5-a]pyrimidine,
2,4-dichloro-6-phenylsulfinylmethylimidazo[1,5-a]pyrimidine,
2,4,8-trichloro-6-phenylimidazo[1,5-a]pyrimidine,
2,4-dichloro-8-iodo-6-(4-methylphenyl)imidazo[1,5-a]pyrimidine,
2,4-dichloro-8-bromo-6-(4-methylphenyl)imidazo[1,5-a]pyrimidine,
2,4,8-trichloro-6-(3,4-dichlorophenyl)imidazo[1,5-a]pyrimidine,
2,4,8-trichloro-6-(4-bromophenyl)imidazo[1,5-a]pyrimidine,
2,4,8-trichloro-6-(4-chlorobenzyl)imidazo[1,5-a]pyrimidine,
2,4,8-trichloro-6-cyclohexylimidazo[1,5-a]pyrimidine,

and their acid addition salts.

Further on another object of the invention is a process for preparing imidazo[1,5-a]pyrimidine derivatives, namely

2,4-dichloro-6-(2-thienyl)imidazo[1,5-a]pyrimidine,
2,4-dichloro-6-(5-phenyl-3-isoxazolyl)imidazo[1,5-a]pyrimidine,
2,4-dichloro-6-(2-furyl)imidazo[1,5-a]pyrimidine,
2,4-dichloro-6-(2-thienylmethyl)imidazo[1,5-a]pyrimidine,
2,4-dichloro-6-(3-thienyl)imidazo[1,5-a]pyrimidine,
2,4-dichloro-6-(5-bromo-2-thienyl)imidazo[1,5-a]pyrimidine,
2,4-dichloro-6-(5-chloro-2-thienyl)imidazo[1,5-a]pyrimidine,
2,4-dichloro-6-(5-methyl-2-thienyl)imidazo[1,5-a]pyrimidine,
2,4-dichloro-6-(3-pyridyl)imidazo[1,5-a]pyrimidine,
2,4-dichloro-6-(3-chloro-1-benzothiophen-2-yl)imidazo[1,5-a]pyrimidine,
2,4-dichloro-6-phenylthiomethylimidazo[1,5-a]pyrimidine,
2,4-dichloro-6-phenylsulfinylmethylimidazo[1,5-a]pyrimidine,

characterized by converting a compound represented by the formula (I)

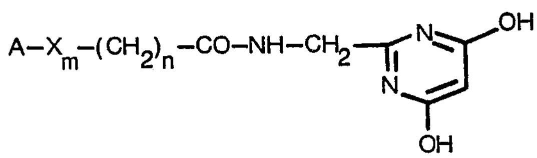

(I)

with a condensing agent such as phosphorous halide and phosphoryl halide, for example phosphorus trichloride, phosporous pentachloride, phosphorous tribromide, phosphoryl chloride and thionyl chloride, wherein X represents a sulfur atom, a sulfinyl group, a sulfonyl group, an oxygen atom m and n are each independently 0 or 1 and A is a phenyl group which may be substituted, a cycloalkyl group or an aromatic heterocyclic group which may be substituted (but when m is 0, A is not a phenyl group which may be substituted or a cycloalkyl group).

A further object of the invention is a process for preparing imidazo[1,5-a]pyrimidine derivatives, namely:

2,4-dichloro-8-iodo-6-(4-methylphenyl)imidazo[1,5-a]pyrimidine,
2,4-dichloro-8-bromo-6-(4-methylphenyl)imidazo[1,5-a]pyrimidine,
2,4,8-trichloro-6-(3,4-dichlorophenyl)imidazo[1,5-a]pyrimidine,
2,4,8-trichloro-6-(4-bromophenyl)imidazo[1,5-a]pyrimidine,
2,4,8-trichloro-6-(4-chlorobenzyl)imidazo[1,5-a]pyrimidine,
2,4,8-trichloro-6-cyclohexylimidazo[1,5-a]pyrimidine,
2,4,8-trichloro-6-phenylimidazo[1,5-a]pyrimidine

characterized by converting the imidazo[1,5-a]pyrimidine derivatives of the formula (II)

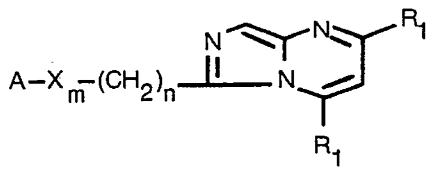

(II)

wherein $R_1$ is a halogen atom, X is a sulfur atom, a sulfinyl group, a sulfonyl group or an oxygen atom, m and n are each independently 0 or 1 and A is a phenyl group, which may be substituted, a cycloalkyl group or an aromatic heterocyclic group, which may be substituted, with a halogenation agent such as N-halosuccinimide.

The manufacture of the compounds of the invention may be summarized by the following reaction scheme:

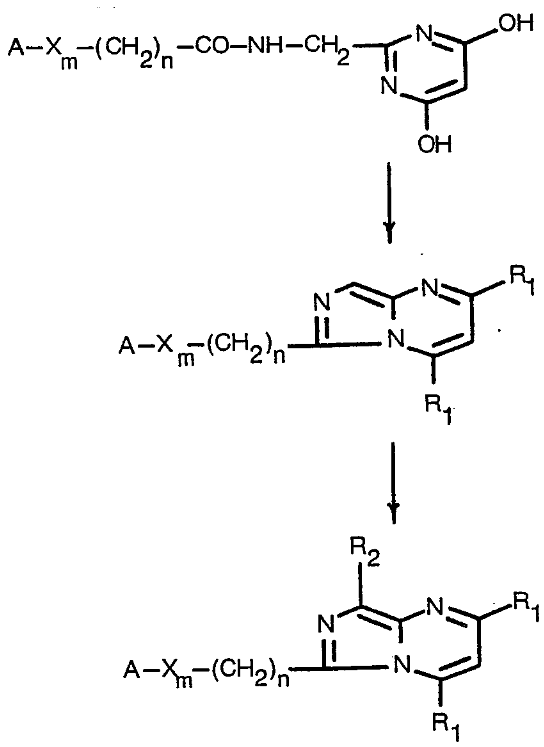

(I)

(II)

wherein $R_1$ is a halogen atom, $R_2$ is a hydrogen or a halogen atom, X is a sulfur atom, a sulfinyl group, a sulfonyl group or an oxygen atom, m and n are each independently 0 or 1, A is a phenyl group which may be substituted, a cycloalkyl group or an aromatic heterocyclic group which may be substituted (but when m is 0 and A is a phenyl group which may be substituted or a cycloalkyl group, $R_2$ is a halogen atom).

(The latter general formula wherein A, X, m, n, $R_1$ and $R_2$ have the meanings as defined above, represents the compounds of the invention).

The N-acylaminomethylpyrimidine compounds represented by the formula (I) were converted to the imidazo[1,5-a]pyrimidine compounds of the formula (II) by the condensing agents such as phosphorous halide and phosphoryl halide, for example phosphorous trichloride, phosphorous pentachloride, phosphorous tribromide, phosphoryl chloride, and thionyl chloride.

The compounds of the formula (II) can be converted to the compounds represented by the general formula by treating with halogenation agent such as N-halosuccinimide for example N-bromosuccinimide, N-chlorosuccinimide. The compound having a thio group in the formula (II) or in the general formula can also be oxidized, for example the thio group to a sulfinyl group or a sulfonyl group by the usual manner.

The above compounds of the invention may be used not only as a medicine for human beings, but also as a drug for animals, fish and shells and antiseptic for food in the various forms.

The suitable salts corresponding to the compounds represented by the general formula may be derived from inorganic acids, such as hydrochloric acid, sulfuric acid or derived from organic acid, such as acetic acid, tartaric acid or methanesulfonic acid etc.

N-acylaminomethylpyrimidine compounds having the formula (I), the starting materials in the reaction discussed above, are also novel compounds and can be prepared by the following methods.

The method for preparing these compounds involves (a) reaction of glycine ethyl ester hydrochloride with various acid chlorides to give N-acyl derivatives of glycine ethyl ester and (b) treatment of these N-acyl derivatives with malonamide to give the desired compounds (I). These reactions are summarized in the following scheme.

$$A{-}X_m{-}(CH_2)_n{-}COCl + NH_2{-}CH_2{-}COOR_3 \cdot HCl \xrightarrow[(a)]{}$$

$$A{-}X_m{-}(CH_2)_n{-}CONHCH_2COOR_3 \xrightarrow[(b)]{CH_2{=}(CONH_2)_2} \quad (I)$$

wherein A, X, m and n have the previously defined meanings, $R_3$ is a lower alkyl group.

Preparation of intermediate compounds:

Reference 1 Preparation of 2-[N-(2-thenoyl)aminomethyl]-4,6-dihydroxypyrimidine

(i) N-(2-thenoyl)glycine ethyl ester

To a solution of glycine ethyl ester hydrochloride (22 g) and potassium carbonate (86 g) in water (600 ml) was added a mixture of benzene (600 ml) and ether (400 ml) and stirred at room temperature. 2-Thenoyl chloride (25 g) in benzene (100 ml) was added during a period of about 30 minutes under stirring and then the mixture was stirred for 2 hours at room temperature. The organic layer was separated and after dried over anhydrous sodium sulfate, concentrated under reduced pressure to give colorless crystals, which were recrystallized from ethanol to obtain the objective compound (23 g), mp 82—83°C.

(ii) 2-[N-(2-thenoyl)aminomethyl]-4,6-dihydroxypyrimidine

To a solution of sodium (1.4 g) in ethanol (120 ml) was added malonamide (3.8 g) and the mixture was heated at 60°C for an hour under stirring. Then after added N-(2-thenoyl)glycine ethyl ester (8 g), the mixture was refluxed for 6 hours. The mixture was concentrated to give the residue which was diluted with water. The aqueous mixture was neutralized by adding acetic acid to deposit crystalline product which was collected by filtration and recrystallized from dimethylformamide (DMF) to give the objective product (2.8 g) as colorless crystals, mp 270—275°C (decompd.).

Analysis (%) for $C_{10}H_9N_3O_3S$, Calcd. (Found): C, 47.80 (47.83); H, 3.61 (3.67); N, 16.72 (16.96).

Other compounds prepared by the same manner as this example are as follows.

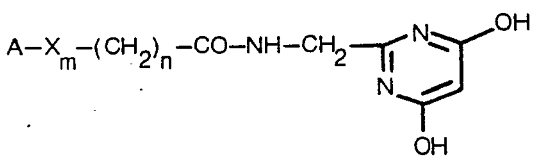

(I)

| Reference Example | A | X | m | n | Formula | mp (°C) |
|---|---|---|---|---|---|---|
| 2 | 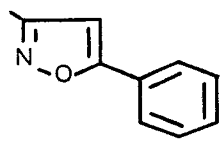 | — | 0 | 0 | $C_{15}H_{12}N_4O_4$ | 220—225 (decompd.) |
| 3 | 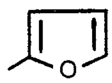 | — | 0 | 0 | $C_{10}H_9N_3O_4$ | 285—290 (decompd.) |
| 4 | 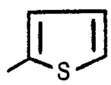 | — | 0 | 1 | $C_{11}H_{11}N_3O_3S$ | 275—285 (decompd.) |
| 5 | 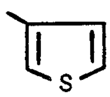 | — | 0 | 0 | $C_{10}H_9N_3O_3S$ | >300 |
| 6 | 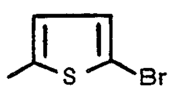 | — | 0 | 0 | $C_{10}H_8N_3O_3BrS$ | 250—255 (decompd.) |
| 7 | 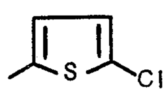 | — | 0 | 0 | $C_{10}H_8N_3O_3ClS$ | >300 |
| 8 | 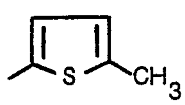 | — | 0 | 0 | $C_{11}H_{11}N_3O_3S$ | 269—276 (decompd.) |
| 9 | 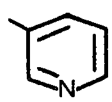 | — | 0 | 0 | $C_{11}H_{10}N_4O_3$ | 255—260 (decompd.) |
| 10 | 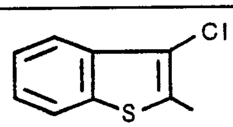 | — | 0 | 0 | $C_{14}H_{10}N_3O_3SCl$ | 185—195 (decompd.) |

## Example 1

2,4-dichloro-6-(2-thienyl)imidazo[1,5-a]pyrimidine

A mixture of 2-[N-(2-thenoyl)aminomethyl]-4,6-dihydroxypyrimidine (1.3 g) and phosphoryl chloride (10 ml) was heated to reflux for 3 hours. The excessive phosphoryl chloride was removed by distillation under reduced pressure. To the resulting residue was added an aqueous sodium carbonate solution and the mixture was extracted with chloroform. The chloroform layer was washed with water, dried over anhydrous sodium sulfate and concentrated. The residue was purified by alumina column chromatography, eluting with benzene and then by recrystallization to give the objective compound (0.56 g) as yellow plates, mp 152—153°C.

Analysis (%) for $C_{10}H_5N_3SCl_2$, Calcd. (Found): C, 44.46 (44.32); H, 1.87 (1.67); N, 15.56 (15.56).

Other compounds prepared by the same manner as this example are as follows.

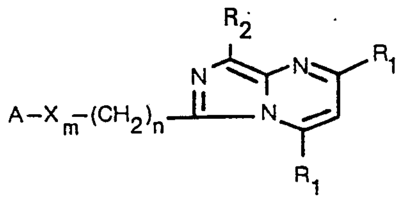

| Example | $R_1$ | $R_2$ | A | X | m | n | Formula | mp (°C) | Analysis (%) Calcd. Found C | H | N |
|---|---|---|---|---|---|---|---|---|---|---|---|
| 2 | Cl | H | 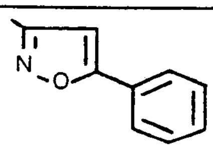 | — | 0 | 0 | $C_{15}H_8N_4OCl_2$ | 149—150 | 54.40<br>54.37 | 2.43<br>2.18 | 16.92<br>16.87 |
| 3 | Cl | H | 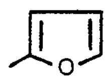 | — | 0 | 0 | $C_{10}H_5N_3OCl_2$ | 116—117 | 47.27<br>47.08 | 1.98<br>1.80 | 16.54<br>16.68 |
| 4 | Cl | H | 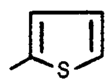 | — | 0 | 1 | $C_{11}H_7N_3Cl_2S$ | 86—87 | 46.49<br>46.21 | 2.48<br>2.30 | 14.79<br>14.92 |
| 5 | Cl | H | 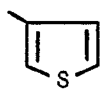 | — | 0 | 0 | $C_{10}H_5N_3Cl_2S$ | 146—147 | 44.46<br>44.25 | 1.87<br>1.68 | 15.56<br>15.68 |
| 6 | Cl | H | 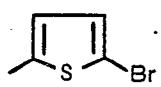 | — | 0 | 0 | $C_{10}H_4N_3Cl_2SB$ | 164—165 | 34.41<br>34.67 | 1.16<br>1.03 | 12.04<br>12.09 |
| 7 | Cl | H | 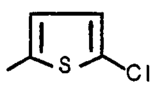 | — | 0 | 0 | $C_{10}H_4N_3Cl_3S$ | 166—167 | 39.43<br>39.29 | 1.32<br>1.16 | 13.80<br>13.99 |
| 8 | Cl | H | 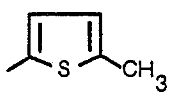 | — | 0 | 0 | $C_{11}H_7N_3Cl_2S$ | 117—118 | 46.49<br>46.39 | 2.48<br>2.36 | 14.79<br>15.00 |
| 9 | Cl | H | 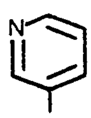 | — | 0 | 0 | $C_{11}H_6N_4Cl_2$ | 140—142<br>(dec.) | 49.84<br>49.81 | 2.28<br>2.11 | 21.13<br>21.32 |
| 10 | Cl | H | 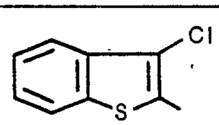 | — | 0 | 0 | $C_{14}H_6N_3Cl_3S$ | 173—175 | 47.42<br>47.41 | 1.71<br>1.53 | 11.85<br>12.00 |
| 11 | Cl | H | 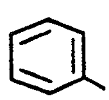 | S | 1 | 1 | $C_{13}H_9N_3Cl_2S$ | 111—112 | 50.34<br>50.41 | 2.92<br>2.79 | 13.55<br>13.60 |
| 12 | Cl | H | 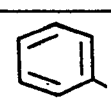 | S=O | 1 | 1 | $C_{13}H_9N_3Cl_2SO$ | 153—154 | 47.87<br>47.89 | 2.78<br>2.69 | 12.88<br>12.80 |

Example 13

2,4,8-trichloro-6-phenylimidazo[1,5-a]pyrimidine

A mixture of 2,4-dichloro-6-phenylimidazo[1,5-a]pyrimidine (0.88 g), N-chlorosuccinimide (0.5 g) and benzoyl peroxide (0.04 g) in carbon tetrachloride (20 ml) was heated to reflux for 14 hours. The mixture was concentrated and purified by alumina column chromatography, eluting with benzene to give the objective product which was recrystallized from ethanol to yellow plates (0.32 g), mp 133—134°C.

Analysis (%) for $C_{12}H_6N_3Cl_3$, Calcd. (Found): C, 48.28 (48.36); H, 2.03 (1.81); N, 14.07 (14.35).

Other compounds prepared by the same manner as this example are as follows.

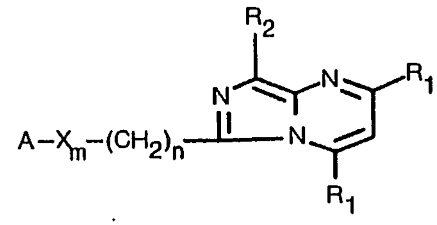

| Example | $R_1$ | $R_2$ | A | X | m | n | Formula | mp (°C) | Analysis (%) Calcd. Found C | H | N |
|---|---|---|---|---|---|---|---|---|---|---|---|
| 14 | Cl | I | 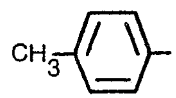 | — | 0 | 0 | $C_{13}H_8N_3Cl_2I$ | 192—193 | 38.65<br>38.65 | 2.00<br>1.90 | 10.40<br>10.39 |
| 15 | Cl | Br | 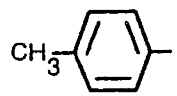 | — | 0 | 0 | $C_{13}H_8N_3BrCl_2$ | 194—195 | 43.73<br>43.49 | 2.26<br>2.13 | 11.77<br>11.27 |
| 16 | Cl | Cl | 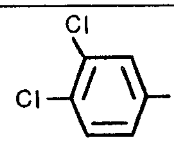 | — | 0 | 0 | $C_{12}H_4N_3Cl_5$ | 186—187 | 39.23<br>39.29 | 1.10<br>0.93 | 11.44<br>11.57 |
| 17 | Cl | Cl | 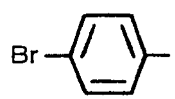 | — | 0 | 0 | $C_{12}H_5N_3BrCl_3$ | 182—183 | 38.19<br>38.25 | 1.34<br>1.12 | 11.13<br>11.15 |
| 18 | Cl | Cl | 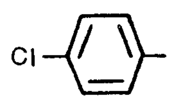 | — | 0 | 1 | $C_{13}H_7N_3Cl_4$ | 116—117 | 44.99<br>45.12 | 2.03<br>1.90 | 12.01<br>12.03 |
| 19 | Cl | Cl | 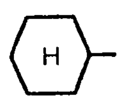 | — | 0 | 0 | $C_{12}H_{12}N_3Cl_3$ | 153—154 | 47.32<br>47.32 | 3.97<br>3.82 | 13.79<br>13.94 |

Experiment 1 Antifungal spectra

The antifungal activity of the compound of the present invention was assayed by the standard agar dilution streak method against fungi. The results were shown in Table 1. M.I.C. studied with representative members of the compound of this invention have demonstrated extremely favourable antimycotic activity, so that these compounds will be very useful as therapeutic for agents, drugs for animals, fishes and shells, and an antiseptic for food.

Antifungal Activity

| Organism | Minimum inhibitory concentration (µg/ml) | | | | | | | | | | |
|---|---|---|---|---|---|---|---|---|---|---|---|
| | Exp. 1 | Exp. 3 | Exp. 4 | Exp. 5 | Exp. 6 | Exp. 7 | Exp. 8 | Exp. 13 | Exp. 17 | Exp. 18 | Exp. 19 |
| Candida albicans 3147 | 3.13 | 3.13 | 3.13 | 3.13 | 0.78 | 0.20 | 1.56 | 3.13 | 1.56 | 1.56 | 0.78 |
| Candida albicans IFO—1388 | 1.56 | 3.13 | 6.25 | 6.25 | 0.78 | 0.78 | 6.25 | 3.13 | 0.78 | 0.20 | 0.20 |
| Candida albicans IFO—1594 | 1.56 | 3.13 | 3.13 | 3.13 | 3.13 | 0.20 | 6.25 | 3.13 | 1.56 | 3.13 | 1.56 |
| Candida albicans MTU—12124 | 6.25 | 6.25 | 6.25 | 6.25 | 0.78 | 1.56 | 3.13 | 12.5 | 3.13 | 6.25 | 1.56 |
| Candida albicans KYF—602 | 6.25 | 12.5 | 6.25 | 12.5 | 3.13 | 0.39 | 6.25 | 3.13 | 6.25 | 25 | 1.56 |
| Candida stellatidea IFO—1398 | 1.56 | 3.13 | 3.13 | 6.25 | 0.78 | 0.78 | 1.56 | 6.25 | 0.39 | 0.78 | 1.56 |
| Microsporum canis 200100 | 1.56 | 1.56 | 1.56 | 1.56 | 0.78 | — | — | 0.78 | — | — | — |
| Aspergillus fumigatus MTU—06002 | 6.25 | 12.5 | 12.5 | 3.13 | 3.13 | — | — | 6.25 | — | — | — |
| Trichophyton mentagrophytes MTU—19003 | 1.56 | 3.13 | 1.56 | 1.56 | 1.56 | 0.78 | 1.56 | 0.78 | 3.13 | 3.13 | 3.13 |
| Trichophyton mentagrophytes MTU—19005 | 1.56 | 3.13 | 1.56 | 0.78 | 0.78 | 0.78 | 1.56 | 0.78 | 3.13 | 1.56 | 1.56 |

Experiment 2 In vivo antifungal activity against systemic infection in mice (ICR)

Mouse was infected intraperitoneal with Candida albicans. (Strain KYF—1385, challenge dose 2.6—7.2 × $10^6$ cfu/mouse, n=5) After the infection, compounds were treated with 100 mg/kg/day twice a day during 4 days, oral administration.

The efficacy of the compound of the invention are shown in Figure together with control (no treated). The present compounds were more effective than the control.

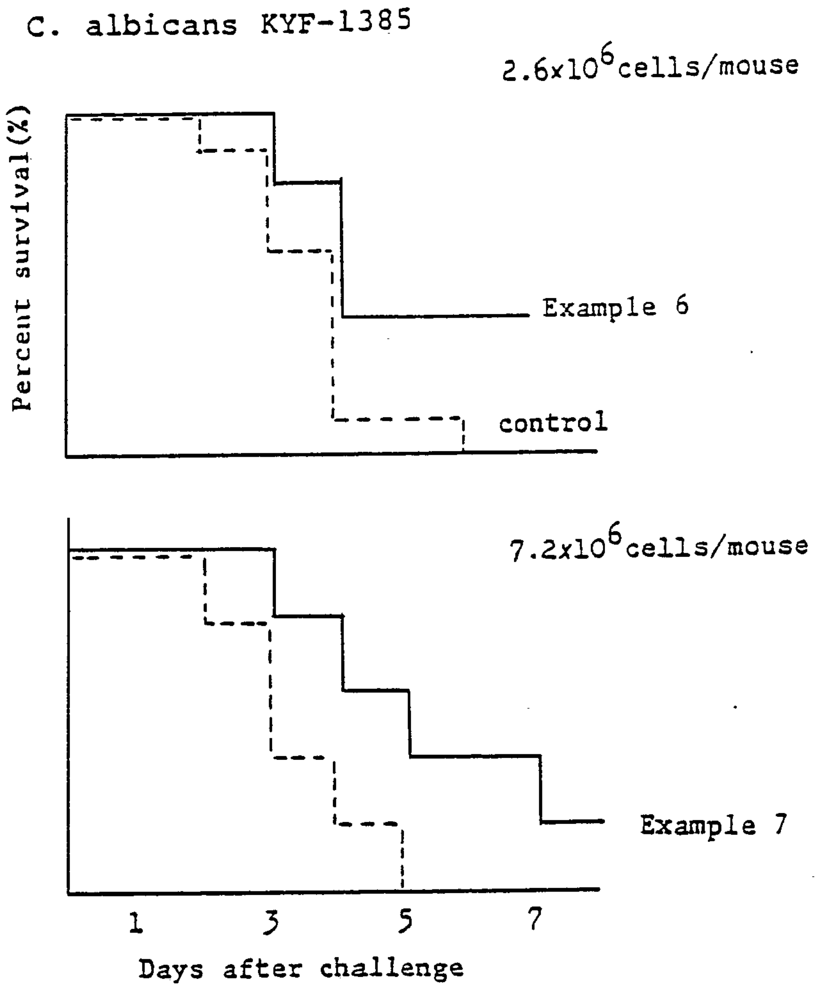


Effect of present compound on murine systemic candidosis by oral administration

**Claims**

1. Imidazo[1,5-*a*]pyrimidine derivatives, namely:
2,4-dichloro-6-(2-thienyl)imidazo[1,5-a]pyrimidine,
2,4-dichloro-6-(5-phenyl-3-isoxazolyl)imidazo[1,5-a]pyrimidine,
2,4-dichloro-6-(2-furyl)imidazo[1,5-a]pyrimidine,
2,4-dichloro-6-(2-thienylmethyl)imidazo[1,5-a]pyrimidine,
2,4-dichloro-6-(3-thienyl)imidazo[1,5-a]pyrimidine,
2,4-dichloro-6-(5-bromo-2-thienyl)imidazo[1,5-a]pyrimidine,
2,4-dichloro-6-(5-chloro-2-thienyl)imidazo[1,5-a]pyrimidine,
2,4-dichloro-6-(5-methyl-2-thienyl)imidazo[1,5-a]pyrimidine,
2,4-dichloro-6-(3-pyridyl)imidazo[1,5-a]pyrimidine,
2,4-dichloro-6-(3-chloro-1-benzothiophen-2-yl)imidazo[1,5-a]pyrimidine,
2,4-dichloro-6-phenylthiomethylimidazo[1,5-a]pyrimidine,
2,4-dichloro-6-phenylsulfinylmethylimidazo[1,5-a]pyrimidine,
2,4,8-trichloro-6-phenylimidazo[1,5-a]pyrimidine,
2,4-dichloro-8-iodo-6-(4-methylphenyl)imidazo[1,5-a]pyrimidine,
2,4-dichloro-8-bromo-6-(4-methylphenyl)imidazo[1,5-a]pyrimidine,
2,4,8-trichloro-6-(3,4-dichlorophenyl)imidazo[1,5-a]pyrimidine,
2,4,8-trichloro-6-(4-bromophenyl)imidazo[1,5-a]pyrimidine,

2,4,8-trichloro-6-(4-chlorobenzyl)imidazo[1,5-a]pyrimidine,
2,4,8-trichloro-6-cyclohexylimidazo[1,5-a]pyrimidine,
and their acid addition salts.

2. An antimycotic agent comprising a compound as claimed in claim 1.

3. A process for preparing imidazo[1,5-a]pyrimidine derivatives, namely
2,4-dichloro-6-(2-thienyl)imidazo[1,5-a]pyrimidine,
2,4-dichloro-6-(5-phenyl-3-isoxazolyl)imidazo[1,5-a]pyrimidine,
2,4-dichloro-6-(2-furyl)imidazo[1,5-a]pyrimidine,
2,4-dichloro-6-(2-thienylmethyl)imidazo[1,5-a]pyrimidine,
2,4-dichloro-6-(3-thienyl)imidazo[1,5-a]pyrimidine,
2,4-dichloro-6-(5-bromo-2-thienyl)imidazo[1,5-a]pyrimidine,
2,4-dichloro-6-(5-chloro-2-thienyl)imidazo[1,5-a]pyrimidine,
2,4-dichloro-6-(5-methyl-2-thienyl)imidazo[1,5-a]pyrimidine,
2,4-dichloro-6-(3-pyridyl)imidazo[1,5-a]pyrimidine,
2,4-dichloro-6-(3-chloro-1-benzothiophen-2-yl)imidazo[1,5-a]pyrimidine,
2,4-dichloro-6-phenylthiomethylimidazo[1,5-a]pyrimidine,
2,4-dichloro-6-phenylsulfinylmethylimidazo[1,5-a]pyrimidine,
characterized by converting a compound represented by the formula (I)

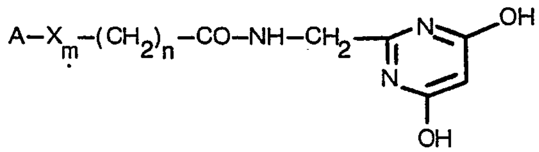

(I)

with a condensing agent such as phosphoryl chloride or thionyl chloride wherein X represents a sulfur atom, a sufinyl group, a sulfonyl group, an oxygen atom, m and n are each independently 0 or 1 and A is a phenyl group which may be subsituted, a cylcoalkyl group or an aromatic heterocyclic group which may be substituted (but when m is 0, A is not a phenyl group which may be substituted or a cycloalkyl group).

4. A process for preparing imidazo[1,5-a]pyrimidine derivatives, namely:
2,4-dichloro-8-iodo-6-(4-methylphenyl)imidazo[1,5-a]pyrimidine,
2,4-dichloro-8-bromo-6-(4-methylphenyl)imidazo[1,5-a]pyrimidine,
2,4,8-trichloro-6-(3,4-dichlorophenyl)imidazo[1,5-a]pyrimidine,
2,4,8-trichloro-6-(4-bromophenyl)imidazo[1,5-a]pyrimidine,
2,4,8-trichloro-6-(4-chlorobenzyl)imidazo[1,5-a]pyrimidine,
2,4,8-trichloro-6-cyclohexylimidazo[1,5-a]pyrimidine,
2,4,8-trichloro-6-phenylimidazo[1,5-a]pyrimidine
characterized by converting the imidazo[1,5-a]pyrimidine derivatives of the formula (II)

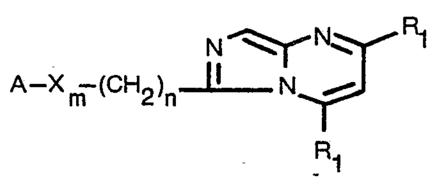

(II)

wherein $R_1$ is a halogen atom, X is a sulfur atom, a sulfinyl group, a sulfonyl group or an oxygen atom, m and n are each independently 0 or 1 and A is a phenyl group, which may be substituted, a cycloalkyl group or an aromatic heterocyclic group, which may be substituted, with a halogenation agent such as N-halosuccinimide.

**Patentansprüche**

1. Imidazo[1,5-*a*]pyrimidin-Derivative, nämlich
2,4-Dichlor-6-(2-thienyl)-imidazo[1,5-a]pyrimidin,
2,4-Dichlor-6-(5-phenyl-3-isoxazolyl)-imidazo[1,5-a]pyrimidin,
2,4-Dichlor-6-(2-furyl)-imidazo[1,5-a]pyrimidin,
2,4-Dichlor-6-(2-thienylmethyl)-imidazol[1,5-a]pyrimidin,
2,4-Dichlor-6-(3-thienyl)-imidazo[1,5-a]pyrimidin,
2,4-Dichlor-6-(5-brom-2-thienyl)-imidazo[1,5-a]pyrimidin,
2,4-Dichlor-6-(5-chlor-2-thienyl)-imidazo[1,5-a]pyrimidin,
2,4-Dichlor-6-(5-methyl-2-thienyl)-imidazo[1,5-a]pyrimidin,
2,4-Dichlor-6-(3-pyridyl)-imidazo[1,5-a]pyrimidin,
2,4-Dichlor-6-(3-chlor-1-benzothiophen-2-yl)-imidazo[1,5-a]pyrimidin,
2,4-Dichlor-6-phenylthiomethylimidazo[1,5-a]pyrimidin,
2,4-Dichlor-6-phenylsulfinylmethylimidazo[1,5-a]pyrimidin,

2,4-8-Trichlor-6-phenylimidazo[1,5-a]pyrimidin,
2,4-Dichlor-8-iod-6-(4-methylphenyl)-imidazo[1,5-a]pyrimidin,
2,4-Dichlor-8-brom-6-(4-methylphenyl)-imidazo[1,5-a]pyrimidin,
2,4,8-Trichlor-6-(3,4-dichlorphenyl)-imidazo[1,5-a]pyrimidin,
2,4,8-Trichlor-6-(4-bromphenyl)-imidazo[1,5-a]pyrimidin,
2,4,8-Trichlor-6-(4-chlorbenyl)-imidazo[1,5-a]pyrimidin,
2,4,8-Trichlor-6-cyclohexylimidazo[1,5-a]pyrimidin,
und ihre Säureadditionssalze.

2. Antimykotisches Mittel, dadurch gekennzeichnet, daß es eine Verbindung nach Anspruch 1 enthält.

3. Verfahren zur Herstellung von Imidazo[1,5-a]pyrimidin-Derivativen, nämlich
2,4-Dichlor-6-(2-thienyl)-imidazo[1,5-a]pyrimidin,
2,4-Dichlor-6-(5-phenyl-3-isoxazolyl)-imidazo[1,5-a]pyrimidin,
2,4-Dichlor-6-(2-furyl)-imidazo[1,5-a]pyrimidin,
2,4-Dichlor-6-(2-thienylmethyl)-imidazo[1,5-a]pyrimidin,
2,4-Dichlor-6-(3-thienyl)-imidazo[1,5-a]pyrimidin,
2,4-Dichlor-6-(5-brom-2-thienyl)-imidazo[1,5-a]pyrimidin,
2,4-Dichlor-6-(5-chlor-2-thienyl)-imidazo[1,5-a]pyrimidin,
2,4-Dichlor-6-(5-methyl-2-thienyl)-imidazo[1,5-a]pyrimidin,
2,4-Dichlor-6-(3-pyridyl)-imidazo[1,5-a]pyrimidin,
2,4-Dichlor-6-(3-chlor-1-benzothiophen-2-yl)-imidazo[1,5-a]pyrimidin,
2,4-Dichlor-6-phenylthiomethylimidazo[1,5-a]pyrimidin,
2,4-Dichlor-6-phenylsulfinylmethylimidazo[1,5-a]pyrimidin,
dadurch gekennzeichnet, daß eine Verbindung der Formel (I)

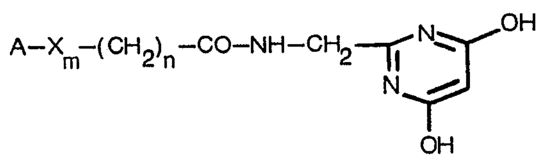

(I)

in der X ein Schwefelatom, eine Sulfinylgruppe-, eine Sulfonylgruppe, ein Sauerstoffatom, m und n unabhängig voneinander 0 oder 1 und A eine Phenylgruppe, die substituiert sein kann, eine Cycloalkylgruppe oder eine aromatische heterocyclische Gruppe, die substituiert sein kann, mit der Maßgabe bedeuten, daß wenn m gleich 0 ist, A keine Phenylgruppe, die substituiert sein kann, oder keine Cycloalkylgruppe darstellt, mit einem Kondensierungsreagens wie Phosphorylchlorid oder Thionylchlorid, umgesetzt wird.

4. Verfahren zur Herstellung von Imidazo[1,5-a]pyrimidin-Derivativen, nämlich
2,4-Dichlor-8-iod-6-(4-methylphenyl)-imidazo[1,5-a]pyrimidin,
2,4-Dichlor-8-brom-6-(4-methylphenyl)-imidazo[1,5-a]pyrimidin,
2,4,8-Trichlor-6-(3,4-dichlorphenyl)-imidazo[1,5-a]pyrimidin,
2,4,8-Trichlor-6-(4-bromphenyl)-imidazo[1,5-a]pyrimidin,
2,4,8-Trichlor-6-(4-chlorbenzyl)-imidazo[1,5-a]pyrimidin,
2,4,8-Trichlor-6-cyclohexylimidazo[1,5-a]pyrimidin,
2,4,8-Trichlor-6-phenylimidazo[1,5-a]pyrimidin,
dadurch gekennzeichnet, daß Imidazo[1,5-a]pyrimidin-Derivate der Formel (II)

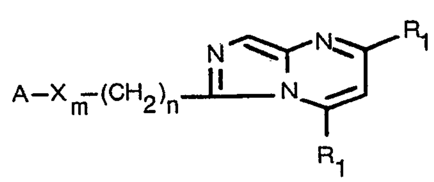

(II)

in der $R_1$ ein Halogenatom, X ein Schwefelatom, eine Sulfinylgruppe, eine Sulfonylgruppe oder ein Sauerstoffatom, m und n unabhängig voneinander 0 oder 1 und A eine Phenylgruppe, die substituiert sein kann, eine Cycloalkylgruppe oder eine aromatische heterocyclische Gruppe, die substituiert sein kann, bedeuten, mit einem Halogenierungsreagens wie N-Haloscuccinimid umgesetzt werden.

**Revendications**

1. Dérivés d'imidazo[1,5-a]pyrimidine, à savoir:
la 2,4-dichloro-6-(2-thiényl)imidazo[1,5-a]pyrimidine,
la 2,4-dichloro-6-(5-phényl-3-isoxazolyl)imidazo[1,5-a]pyrimidine,
la 2,4-dichloro-6-(2-furyl)imidazo[1,5-a]pyrimidine,
la 2,4-dichloro-6-(2-thiénylméthyl)imidazo[1,5-a]pyrimidine,
la 2,4-dichloro-6-(3-thiényl)imidazo[1,5-a]pyrimidine,

la 2,4-dichloro-6-(5-bromo-2-thiényl)imidazo[1,5-a]pyrimidine,
la 2,4-dichloro-6-(5-chloro-2-thiényl)imidazo[1,5-a]pyrimidine,
la 2,4-dichloro-6-(5-méthyl-2-thiényl)imidazo[1,5-a]pyrimidine,
la 2,4-dichloro-6-(3-pyridyl)imidazo[1,5-a]pyrimidine,
la 2,4-dichloro-6-(3-chloro-1-benzothiophène-2-yl)imidazo[1,5-a]pyrimidine,
la 2,4-dichloro-6-phénylthiométhylimidazo[1,5-a]pyrimidine,
la 2,4-dichloro-6-phénylsulfinylméthylimidazo[1,5-a]pyrimidine,
la 2,4,8-trichloro-6-phénylimidazo[1,5-a]pyrimidine,
la 2,4-dichloro-8-iodo-6-(4-méthylphényl)imidazo[1,5-a]pyrimidine,
la 2,4-dichloro-8-bromo-6-(4-méthylphényl)imidazo[1,5-a]pyrimidine,
la 2,4,8-trichloro-6-(3,4-dichlorophényl)imidazo[1,5-a]pyrimidine,
la 2,4,8-trichloro-6-(4-bromophényl)imidazo[1,5-a]pyrimidine,
la 2,4,8-trichloro-6-(4-chlorobenzyl)imidazo[1,5-a]pyrimidine,
la 2,4,8-trichloro-6-cyclohexylimidazo[1,5-a]pyrimidine,
et leurs sels d'addition d'acides.

2. Agent antimycosique comprenant un composé tel que revendiqué dans la revendication 1.

3. Procédé pour la préparation de dérivés d'imidazo[1,5-a]pyrimidine, à savoir:
la 2,4-dichloro-6-(2-thiényl)imidazo[1,5-a]pyrimidine,
la 2,4-dichloro-6-(5-phényl-3-isoxazolyl)imidazo[1,5-a]pyrimidine,
la 2,4-dichloro-6-(2-furyl)imidazo[1,5-a]pyrimidine,
la 2,4-dichloro-6-(2-thiénylméthyl)imidazo[1,5-a]pyrimidine,
la 2,4-dichloro-6-(3-thiényl)imidazo[1,5-a]pyrimidine,
la 2,4-dichloro-6-(5-bromo-2-thiényl)imidazo[1,5-a]pyrimidine,
la 2,4-dichloro-6-(5-chloro-2-thiényl)imidazo[1,5-a]pyrimidine,
la 2,4-dichloro-6-(5-méthyl-2-thiényl)imidazo[1,5-a]pyrimidine,
la 2,4-dichloro-6-(3-pyridyl)imidazo[1,5-a]pyrimidine,
la 2,4-dichloro-6-(3-chloro-1-benzothiophène-2-yl)imidazo[1,5-a]pyrimidine,
la 2,4-dichloro-6-phénylthiométhylimidazo[1,5-a]pyrimidine,
la 2,4-dichloro-6-phénylsulfinylméthylimidazo[1,5-a]pyrimidine,
caractérisé en ce que l'on convertit un composé représenté par la formule (I)

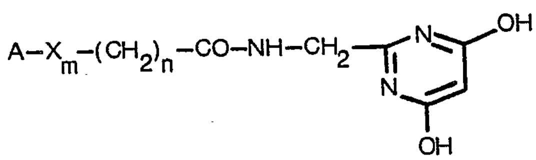
(I)

dans lequelle X représente un atome de soufre, un groupe sulfinyle, un groupe sulfonyle ou un atome d'oxygène, m et n sont chacun indépendamment 0 ou 1, et A est un groupe phényle qui peut être substitué, un groupe cycloalkyle ou un groupe aromatique hétérocyclique qui peut être substitué (mais dans le cas où m est 0, A n'est pas un groupe phényle qui peut être substitué ou un groupe cycloalkyle) avec un agent de condensation tel que le chlorure de phosphoryle ou le chlorure de thionyle.

4. Procédé pour la préparation de dérivés d'imidazo[1,5-a]pyrimidine, à savoir:
la 2,4-dichloro-8-iodo-6-(4-méthylphényl)imidazo[1,5-a]pyrimidine,
la 2,4-dichloro-8-bromo-6-(4-méthylphényl)imidazo[1,5-a]pyrimidine,
la 2,4,8-trichloro-6-(3,4-dichlorophényl)imidazo[1,5-a]pyrimidine,
la 2,4,8-trichloro-6-(4-bromophényl)imidazo[1,5-a]pyrimidine,
la 2,4,8-trichloro-6-(4-chlorobenzyl)imidazo[1,5-a]pyrimidine,
la 2,4,8-trichloro-6-cyclohexylimidazo[1,5-a]pyrimidine,
la 2,4,8-trichloro-6-phénylimidazo[1,5-a]pyrimidine,
caractérisé en ce que l'on convertit les dérivés d'imidazo[1,5-a]pyrimidine de la formule (II)

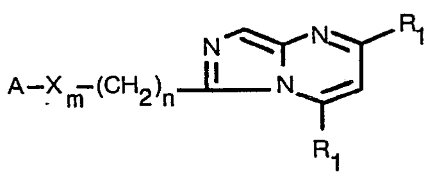
(II)

dans laquelle $R_1$ est un atome d'halogène, X est un atome de soufre, un groupe sulfinyle, un groupe sulfonyle ou un atome d'oxygène, m et n sont chacun indépendamment 0 ou 1 et A est un groupe phényle qui peut être substitué, un groupe cycloalkyle ou un groupe aromatique hétérocyclique qui peut être substitué, avec un agent d'halogénation tel qu'un N-halogénosuccinimide.